(19) Europäisches Patentamt
European Patent Office
Office européen des brevets

(11) Publication number: **0 174 827**
**B1**

(12) **EUROPEAN PATENT SPECIFICATION**

(45) Date of publication of patent specification: **11.07.90**

(51) Int. Cl.$^5$: **D 01 F 9/08** // A61F2/00

(21) Application number: **85306409.5**

(22) Date of filing: **10.09.85**

(54) **Fibrous apatite material and method for producing the same.**

(30) Priority: **11.09.84 JP 190413/84**
**14.09.84 JP 193159/84**
**31.10.84 JP 229283/84**
**31.10.84 JP 229284/84**
**26.02.85 JP 35262/85**
**26.02.85 JP 35263/85**

(43) Date of publication of application:
**19.03.86 Bulletin 86/12**

(45) Publication of the grant of the patent:
**11.07.90 Bulletin 90/28**

(84) Designated Contracting States:
**BE CH DE FR GB IT LI NL SE**

(56) References cited:
**EP-A-0 146 398**
**DE-A-2 652 611**
**GB-A-1 360 197**

**CHEMICAL ABSTRACTS, vol. 88, no. 5, 30th January 1978, page 222, abstract no. 34181s, Columbus, Ohio, US; E. BANKS et al.: "Fibrous apatite grown on modified collagen", & SCIENCE 1977, 198(4322), 1164-6**

(73) Proprietor: **TOA NENRYO KOGYO KABUSHIKI KAISHA**
**1-1 Hitotsubashi, 1-Chome Chiyoda-Ku**
**Tokyo 100 (JP)**

(72) Inventor: **Fujii, Shigeo**
**15-2, Uwadoshinmachi**
**Kawagoe-shi Saitama-ken (JP)**
Inventor: **Mori, Shoichi**
**1902-5, Oaza-Kamekubo Oi-machi**
**Iruma-gun Saitama-ken (JP)**
Inventor: **Tabuchi, Jyoichi**
**1902-5, Oaza-Kamekubo Oi-machi**
**Iruma-gun Saitama-ken (JP)**

(74) Representative: **Harvey, David Gareth et al**
**Graham Watt & Co. Riverhead**
**Sevenoaks Kent TN13 2BN (GB)**

(56) References cited:
**CHEMICAL ABSTRACTS, vol. 97, no. 22, 29th November 1982, page 393, abstract no. 188335t, Columbus, Ohio, US; & JP-A-82 117 621 (MITSUBISHI MINING AND CEMENT CO., LTD) 22-07-1982**

**CHEMICAL ABSTRACTS, vol. 99, no. 10, 5th September 1983, page 371, abstract no. 76915s, Columbus, Ohio, US; & JP-A-58 04 821 (MITSUBISHI MINING AND CEMENT CO., LTD) 12-01-1983**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 174 827 B1**

**Description**

This invention relates to an apatite material in fibrous form and a method for producing it, and more particularly to apatite materials in cotton-like and non-woven fabric form. Such materials have many applications, such as for medical treatment uses, and large scale microorganism cultivating media. In particular, the invention relates to apatite implant materials having good compatibility with living organisms and high working efficiency.

Artificial implant materials conventionally used for remedying breaks or voids made in bones or tooth roots by excision include the patient's own bones, similar bones provided by close relatives, other dissimilar bones, and metallic, organic and carbon materials. However, if a patient's own bone is to be used, the patient can suffer severe pain because a bone organization must be cut from a location other than the affected part. In addition, there may not always be sufficient bone available to provide an amount adequate for the remedy, so that often a substitute is needed to make up the shortage. In order to utilize similar or dissimilar bones other than a patient's own as a substitute, a surgical operation has to be performed on another living organism, which imposes a heavy burden on the bone donor.

Metallic implant materials not only lack affinity with living organism, but also have the disadvantage that metallic ions will plate out of the implant material into the human body, resulting in deterioration of the material. Such metallic ions may also be poisonous. For these reasons, metallic implant materials have proven unsuitable for permanent use. This is also true of organic and carbon materials.

In an attempt to overcome the aforesaid disadvantages single-crystal or polycrystal alumina, silica, alumina or calcium phosphate-based glass, and ceramics such as apatite (see Japanese Patent Application Public Disclosure No. 52-64199 and its counterpart DE—A—2 652 611, for example) have recently been proposed for use as implant materials. Composite materials comprising a metallic core flame spray coated with hydroxy-apatite powders are also known as disclosed in Japanese Patent Application Public Disclosure No. 52-82893. These ceramic materials are superior to other materials because they have a high affinity with living organism and provide direct and intimate connection with bone organization. In particular, hydroxy-apatite is known to be a main inorganic ingredient of a bone or tooth. In this regard, calcined synthetic hydroxy-apatite has drawn increasing attention as a so-called artificial implant material for artificial tooth, bone and the like because it exhibits such good affinity with bone and tooth organizations as to bond directly and chemically with bone organization and gums. (See, for example, Chemistry and Industry, Vol. 37, No. 4, P. 243, 1984). Artificial tooth roots of calcined apatite and artificial bones of porous apatite have reached the stage of practical use.

However, calcined apatite is a brittle material which is vulnerable to tension, although highly resistant to compression, and such apatite tends to have its tensile strength greatly reduced if a hair crack should develop on the surface of the apatite due to a shock, for example. This narrows down the spectrum of applications of calcined apatite to the living organism. The use of the material has thus been limited only to artificial tooth roots for molars or other teeth or the like where no excessive tensile stress will be exerted. Moreover, when such material is to be used as fillers for breaks in bone, difficulties are involved in shaping the material to conform with the intricate contour of the affected part.

In an attempt to eliminate the shortcomings of the calcined apatite described above, Japanese Patent Application Public Disclosures No. 57-117621 and No. 58-54023 discloses inorganic apatite in which the apatite is made fibrous so as to suit it for use as an implant material for breaks or voids in bone. However, the apatite fiber as disclosed in these disclosures is fabricated by the so-called melt spinning process involving the steps of melting apatite and spinning it. As stated also in these disclosures, the melt spinning process requires that the apatite be melted at a high temperature, of 1500°C. As a result, the apatite is deprived of its hydroxy group, and hence its "affinity". The apatite fiber thus has a serious disadvantage in that it does not provide adequate compatibility with the living organism in contrast to hydroxy-apatite. For this reason, such melt spun apatite fiber required a post-treatment for providing it with the required "affinity".

If apatite is to be made fibrous without being deprived of the hydroxy group, the melt spinning process cannot be employed, but another method such as the solution spinning process should be taken into consideration. However, since no binder or no spinning or calcining method suitable for use with the solution spinning process has been developed, it has heretofore been impossible to make apatite in fibrous form, particularly in cotton-like or fabric form with the hydroxy group retained as such.

After extensive research and studies with a view to overcoming the prior art shortcomings as described above, the inventors of the present invention have discovered that it is possible to manufacture fibrous apatite, particularly cotton-like apatite and non-woven fabric thereof by solution spinning apatite with the use of special binder to make the apatite in fibrous form, particularly in cotton-like and non-woven fabric form, and calcining the thus made apatite.

The invention accordingly aims to provide apatite material in fibrous form, particularly in cotton-like and non-woven fabric form suitable, inter alia, for medical treatment uses and large scale microorganism cultivating media.

Another aim is to provide an hydroxy-apatite material in this fibrous form, which has excellent compatibility with the living organism, superior physical properties such as improved tensile strength and the like, and a high workability.

The invention further aims to provide an efficient method of producing the apatite material of the foregoing types.

Fibrous apatites are disclosed in *Science*, 1977, *198*, pp 1164—1166. The product is prepared by enzymatic hydrolysis of Ca β-glycerophosphate on modified collagen (a process wholly dissimilar to that taught herein), and the fibers are in the form of bundles of *parallel* fibers.

The present invention, in contrast, provides a fibrous apatite product, such as calcium apatite, characterised in that the product has an intertwined fibrous, cotton-like form and is made of one or more apatites selected from those represented by the general formula:

$$M_{10}(ZO_4)_6X_2$$

wherein M represents Ca, Ba, Mg, Sr, Pb, Cd and Fe, $ZO_4$ represents $PO_4$, $AsO_4$, $VO_4$ and $CO_3$, and X represents F, Cl and OH.

The fibrous product of apatite may have calcium phosphate type compound(s) incorporated therein for reinforcement.

A fibrous product of apatite according to this invention may be manufactured in the form of non-woven fabric if desired.

Production of the fibrous apatite according to the invention can be accomplished by a method similar to that disclosed in GB—A—1,360,197. Therefore, the present invention also provides a method of producing a fibrous product of apatite of the type defined above, comprising the steps of preparing a dispersion of fine apatite particles with the use of a water-soluble high molecular compound, and spinning this dispersion by solution spinning process. Thus, according to the present invention, there is provided a method of producing the above-defined apatite, comprising the steps of:

(a) preparing a dispersion of one or more of the selected apatites by dispersing fine apatite particles uniformly in a solution of binder in water, the binder for example being a water soluble high molecular weight compound such as pollulan;

(b) continuously extruding the dispersion through a plurality of spinning orifices while simultaneously stretching the dispersion into a fibrous state with the aid of a high speed air flow to form a stream of fine fibers;

(c) heating the fiber stream to evaporate the water in the fibers, to dewater the stream to a moisture content of 10% or less;

(d) blowing the so dewatered fiber stream onto a collector means thereby to form a cotton-like product of intertwined fibrous apatite bonded by the binder; and

(e) optionally, if required, calcining the cotton-like product.

The invention will be more fully understood from the following detailed description which is given by way of example only, taken with reference to the accompanying drawings, in which:

Fig. 1 is a schematic side elevational view of one form of apparatus used for producing a cotton-like product of apatite according to this invention;

Fig. 2 is a schematic side elevational view of one form of apparatus used for manufacturing non-woven fabric of apatite according to this invention;

Fig. 3 is an X-ray diffraction pattern of a cotton-like product of hydroxy-apatite according to this invention;

Fig. 4 is an X-ray diffraction pattern of the cotton-like product of hydroxy-apatite shown in Fig. 3 prior to being calcined; and

Fig. 5 is electron micrographs of a cotton-like product of apatite according to this invention prior to and after being calcined, respectively.

According to this invention a fibrous product of apatite, in cotton-like form or non-woven fabric form comprises one or more apatites selected from apatites represented by the general formula:

$$M_{10}(ZO_4)_6X_2.$$

M represents Ca, Ba, Mg, Sr, Pb, Cd, and Fe, $ZO_4$ represents $PO_4$, $AsO_4$, $VO_4$ and $CO_3$, and X represents F, Cl and OH.

The term "cotton-like product" herein used is intended to mean a structure formed by intertwined fibers as if they were cotton. If desired, a cotton-like product of apatite may be made in the form of non-woven fabric.

Any of the apatites as represented by the aforesaid general formula may be selected and employed alone, or in combination with another, to obtain a fibrous product of the apatite or apatites used. When producing medical implant materials, calcium phosphate type apatite is preferably used because of its similarity to bone organization. More preferably, hydroxy-apatite (compound as represented by the aforesaid general formula where M is Ca, $ZO_4$ is $PO_4$ and X is OH) is employed for its beneficial compatibility with the living organism. However, it should be understood that this invention does not preclude the use of such compounds that have members other than Ca, $PO_4$ and OH in the locations of M, $ZO_4$ and X, respectively represented by the aforesaid formula, so long as such other members would not impair the compatability with living organism. In other words, two or more kinds of apatite may be employed.

Particularly preferred is a fibrous product of apatite wherein the apatite is substantially hydroxy-apatite.

Such a fibrous product of apatite, substantially all of which comprises hydroxy-apatite, however, may not be satisfactory with respect to its strength. The fibrous product of apatite may then contain inorganic compound(s) such as a clacium phosphate type compound, for example, to enhance its physical properties such as its strength and the like, and such an apatite product is a preferred embodiment of the invention. The calcium phosphate type compound may be at least one compound selected from calcium hydrogen phosphate ($CaHPO_4$), tri-calcium phos-

phate ($Ca_3(PO_4)_2$), and tetra-calcium phosphate ($Ca_4O(PO_4)_2$). These compounds may be contained in the apatite product generally in an amount of 5 to 95 weight%, preferably 80 weight% or less, more preferably 50 weight% or less. The calcium phosphate compound may be incorporated by initially blending an amount of the compound required into the main ingredient(s), or alternatively by subjecting the hydroxy-apatite to a high temperature treatment to produce such compound from a part of the hydroxy-apatite, as will be described hereinafter.

In a fibrous product according to this invention, the fibers may have generally a mean diameter of 1 µm to 30 µm and a mean length of 1 mm to 1,000 mm. Of course, this is not limitative but just illustrative. As stated before, the fibrous product according to this invention may be made in cotton-like form or non-woven fabric form. For such non-woven fabric the basis weight may generally be in the range of 5 $g/m^2$ to 500 $g/m^2$, but this is not limitative.

A fibrous apatite in cotton-like form of this invention may be produced by the method comprising the steps of:

(a) preparing a dispersion of apatite by dispersing fine apatite particles uniformly in a solution of binder in water, said apatite being one or more of the apatites defined according to the general formula given hereinbefore;

(b) continuously extruding the thus-prepared dispersion through a plurality of spinning orifices in a spinning apparatus while simultaneously stretching the dispersion into a fibrous state with the aid of a high speed air flow to form a stream of fine fibers;

(c) heating the fiber stream to evaporate the water from the fibers to dewater the stream in a moisture content of 10% or less;

(d) blowing the dewatered fiber stream onto a collector means to form a cotton-like product of apatite bonded by the binder; and

(e) if desired, calcining the cotton-like product.

As noted above, for some applications the apatite is preferably a hydroxy-apatite.

Apatite for use in this invention may be either any suitable apatite synthesized in a known manner or any natural one. The fine particles can have a mean particle diameter of 50Å (5 nm) to 1 µm, and preferably 70 to 500Å (7 to 50 nm). In this case, while the configuration of apatite particles is not limited to any particular one, it is preferable to use apatite particles of stick form (i.e. rod-like or acicular) since such particles facilitate the formation of the apatite into fibrous form or cotton-like form. Although the reason for this has not completely been elucidated, it is presumed that it may be easy to orient the stick-like particles in a predetermined direction during the spinning step. Stick-like particles of apatite such as hydroxy-apatite may be prepared by dropping an aqueous solution of phosphoric acid gradually into an alkaline solution (pH: 7 to 11) containing calcium ions, for example.

Binders used to produce a fibrous product of apatite according to this invention may be commonly known water-soluble binders. Such binders may be water-soluble, cotton-like high molecular compounds having —OH, —COOH, —$CONH_2$ groups or the like in their molecules including pullulan which is a linear glucan wherein maltotrioses are bonded by recurring α-1, 6-glycoside linkages, polyvinyl alcohol, polyacrylamide, polyacrylic acid, polymethacrylic acid, polyitaconic acid, polyethylene oxide, polyvinyl pyrolidone, polyvinyl methylene ether, hydroxypropyl cellulose, xanthane gum, guar gum, collagen, hydroxyethyl cellulose, carboxymethylcellulose and the like. While there is no particular limitation as to the molecular weight, such high molecular compounds may have a molecular weight of preferably 20,000 to 2,000,000, and more preferably 50,000 to 1,000,000.

When fibrous products of apatite according to this invention are to be used particularly as medical materials, it is desirable that binders should meet at least two conditions, namely that they be harmless to the living organism and water-soluble. Suitable examples of such binders include high molecular compounds such as polyvinyl alcohol, carboxymethylcellulose, hydroxypropylcellulose, collagen and the like and high molecular polysaccharides such as pullulan, chitin, dextran and the like. Pullulan is particularly suitable.

A feedstock dispersion comprising the apatite particles dispersed in an aqueous solution of the binder as described above is subjected to a spinning step. The composition of the feed dispersion should be in a predetermined range. By way of example, for hydroxy-apatite, its composition may comprise (a) 10 to 90 weight%, preferably 50 to 70 weight%, more preferably 60 to 65% of water, (b) 5 to 70 weight%, preferably 15 to 30 weight%, more preferably 15 to 20 weight% of hydroxy-apatite, and (c) 5 to 40 weight%, preferably 15 to 30 weight%, more preferably 20 to 25 weight% of binder. If the amount of hydroxy-apatite is less than 5 weight%, it would not be able to provide a fibrous apatite material having sufficient strength. Conversely, more than 70 weight% of hydroxy-apatite would undesirably increase the viscosity excessively.

For the purpose of improving the dispersion of hydroxy-apatite in the feedstock liquid, organic carboxylic acid, or plasticizers or softening agents including polyhydric alcohols such as glycerin, sorbitol, maltitol, ethylene glycol, propylene glycol or the like may be added.

Further, inorganic compounds other than apatite, such as a calcium phosphate, for example, may be added in an amount of less than 5% as a dispersing medium to improve the physical properties of fibrous apatite material obtained.

It is preferable to prepare the feedstock liquid described above at a temperature of about 20 to 70°C. The feedstock dispersion is then continuously extruded through spinning orifices while simultaneously a gas is discharged through gas discharge orifice means adjacent to the spinning orifices at a high speed to form the dispersion into a stream of fibers. The fiber stream is rid of water and collected as a fibrous product in cotton-like form, and then, if desired, the product may be calcined.

In case of hydroxy-apatite, it has been experimentally found by X-ray diffraction and infrared spectrophotometry that the fibrous apatite material, if not calcined or if calcined at a temperature lower than 1250°C, will have the structure of $Ca_{10}(PO_4)_6OH_2$ or $Ca_5(PO_4)_3OH$, whereas if calcined at higher than 1250°C, it will be transformed into the structure of $\alpha$-$Ca_3(PO_4)_2$. The transformed product does not retain the hydroxy group, resulting in reduced compatibility with the living organism. In order to retain substantially the structure of hydroxy-apatite, it is preferable to carry out the calcining step at a temperature lower than 1250°C, and preferably lower than 1200°C.

One exemplary method of producing a fibrous product of apatite according to this invention, wherein fine hydroxy-apatite particles are used as a raw material, will now be described with reference to Fig. 1.

First, hydroxy-apatite, binder and water, and if required, dispersant, plasticizer and/or softener are introduced through a feed line 1 into a feedstock tank 2 to prepare a viscous spinning solution. Such spinning solution may be prepared preferably at about 20 to 70°C. This viscous spinning solution is fed through a conduit 15 and then extruded through a spinning nozzle means 5 by a gear pump 4 driven by a motor 3, while simultaneously gas e.g. air is discharged at a high speed through a nozzle means 7 positioned around the spinning nozzle means. The high speed gas flow is produced by a multi-stage blower 6. The spinning nozzle may include a plurality of dies arranged in a straight line in a widthwise direction. A stream 8 of fine fibers stretched and cut in length is formed by discharging gas such as air at about 20 to 60°C from around the spinning nozzle means at a velocity of about 5 to 1000 m/sec. The diameter and length of the fibers may be adjusted so as to be in the ranges of about 1 to 30 µm and 2 to 1,000 mm, respectively by varying the pressure of the gas discharged.

Then, the thus formed stream 8 of fine fibers is heated by a heater 9 such as an infrared heater or the like underlying the spinning nozzel means, to evaporate the water from the fibers to yield fibers with a moisture content of less than 10 weight%, preferably less than about 7 weight% thereby to solidify the fiber stream. It should be noted that excessive removal of water would result in failure to produce a fibrous apatite in cotton-like form composed of fine fibers. In this regard, the temperature of the heater may generally be in the range of about 200 to 500°C for the fiber stream to be at about 80 to 150°C. The heating temperature for the fiber stream is selected depending upon the extrusion rate of the spinning solution, and the temperature and volume of the gas blown. Excessively high heating temperatures are not desirable for they would cause decomposition of the binder during the heating step.

The fine fiber stream 10 as stretched, cut in length and de-watered is then blown onto and thus collected on a suitable collector means 11, which may be in the form of a screen-type collector vessel or a screen-type collecting belt.

If a fibrous apatite in non-woven fabric form is to be manufactured, the fine fiber stream as stretched and dewatered as described above is caused to drop onto a moving collector means in the form of a screen type rotary drum assembly 16 as shown in Fig. 2, or a moving belt conveyor, the fibers being intertwined to form a non-woven fabric. If the stream of fibers is blown down into the nip between the two rotating screen type drums 16, a bulky non-woven fabric 17 having the intertwined fibers oriented in a direction of thickness of the non-woven fabric (that is, in a three-dimensional configuration) will be obtained. In an alternate embodiment, if the fiber stream is blown down onto those areas of the rotating drums other than the nip between the drums or onto a planar collecting belt conveyor, a non-woven fabric will be produced in which the fibers are oriented parallel in a plane (that is, in a two-dimensional manner). The basis weight of the non-woven fabric may be regulated so as to be in the range of 5 g/m$^2$ to 500 g/m$^2$, for example by varying the speed at which the collector means moves.

The thus produced fibrous apatite, namely, cotton-like product or non-woven fabric of apatite fibers bonded together by the binder provides a feeling soft and agreeable to the touch. Further, owing to its hydrophilic nature, moisture-absorption characteristics and non-electrifying property as well as its cultivation promoting effect, such cotton-like product or non-woven fabric of apatite in an uncalcined state may be employed as a culture medium for microorganisms.

Uncalcined cotton-like product or non-woven fabric may be calcined at a temperature of about 500 to 1250°C, preferably about 600 to 1200°C, more preferably 650 to 1100°C to burn the binder off whereby a fibrous apatite material of this invention is obtained. At a calcining temperature lower than 1200°C there is very little loss of hydroxy group, so that no account may be taken of the loss of hydroxy group although it depends somewhat upon the calcining temperature. If required, a calcining temperature higher than 1200°C may be used, to produce $Ca_3(PO_4)_2$ in the hydroxy-apatite and thereby enhance the physical properties such as strength of the cotton-like product or non-woven fabric of hydroxy-apatite when it is for use as implant material.

The thus obtained cotton-like product or non-woven fabric may be subjected to further processing depending on the purpose for which it is to be used. By way of example, collagen may be applied to or impregnated in the product to further improve the desired properties thereof. It may also be combined with a cultivating substance such as pullulan to suit it for use as a large scale culture medium.

A preferred fibrous apatite material contains a calcium phosphate-type compound in addition to hydroxy-apatite, and may be produced by a method comprising the steps of: adding a water-soluble binder to an aqueous solution containing fine particles of hydroxy-apatite and calcium phosphate-type compound to form a viscous dispersion, continuously spinning the dispersion through a plurality of spinning orifices of a spinning machine or spinneret while simultaneously discharging a gas at a high velocity through gas discharge orifice means adjacent to the spinning orifices, to stretch the fibers from the spinning orifices into a stream of fine fibers, heating the fiber stream to remove the water therefrom, and blowing the de-watered fiber stream onto a collector means e.g. in the form of a collecting plate, to form a cotton-like product of apatite.

In this case, the composition of the starting dispersion may preferably comprise 5 to 90 weight% of water, 5 to 70 weight% of apatite, 5 to 70 weight% of calcium phosphate with 2 to 30 weight% of pullulan. A more suitable composition is 25% of water, 35% of hydroxy-apatite, 30% of calcium phosphate, and 10% of pullulan on the basis of weight. The other manufacturing conditions may be the same as for the production of the fibrous product as described hereinbefore substantially all of which comprises apatite.

Examples of the present invention will be dsecribed hereinbelow, it being understood that this is not to limit the invention.

Example 1

11 weight% of pullulan particles having a mean molecular weight of 200,000, 17 weight% of hydroxy-apatite particles having a particle diameter of 50 to 800Å (5 to 80 nm), and 72 weight% of water were kneaded together to disperse the hydroxy-apatite uniformly in the aqueous pullulan solution. The thus prepared solution was defoamed and fed into a spinning apparatus as shown in Fig. 1 at room temperature (25°C). The apparatus was equipped with a die having a plurality of 0.3 mm diameter spinning nozzles arrayed in a straight line in a transverse direction. The solution was extruded through the nozzles at a pressure of 1.4 kg/cm$^2$ while discharging air through the air gaps at a velocity of 250 m/sec thereby to form a stream of fibers. This fiber stream was heated from opposite sides at a temperature of 400°C by a far infrared heater (wavelength: 2 to 50 μm) underlying the spinning nozzles to evaporate the water, and was blown onto a screen type belt to produce a fibrous product in cotton-like form of hydroxy-apatite bonded by pullulan. The thus produced cotton-like product was heated, at a heating rate of 50°C/hr., for calcining at 1100°C for an hour, whereby a cotton-like hydroxy-apatite product having a mean fiber diameter of 5 μm and a mean fiber length of 50 mm was obtained as the pullulan was burned off. It was determined by X-ray diffraction and infrared ray spectrophotometry that the resulting product contained substantially the same amount of hydroxy group as the starting material. Figs. 3 and 4 show X-ray diffraction patterns prior to and after the calcining step, respectively.

Example 2

Pullulan particles with a mean molecular weight of 200,000 were dissolved in water to prepare 21 weight% of aqueous solution into which 6 weight% of hydroxy-apatite particles having a particle diameter of 50 to 300Å (5 to 30 nm) were mixed. The solution was then defoamed. The resulting solution was fed into the apparatus used in the Example 1 at room temperature and extruded at a pressure of 1.4 kg/cm$^2$ while discharging air through the air gaps at a velocity of 500 m/sec, thereby to form a stream of fibers. This fiber stream was heated from opposite sides at a temperature of 400°C by a far-infrared heater (wavelength: 2—50 μm) underlying the spinning nozzles to evaporate the water and was blown onto a screen type belt to produce a fibrous product in cotton-like form of hydroxy-apatite bonded by pullulan. The resulting product was heated, at a heating rate of 50°C/hr., for calcining at 1400°C for two hours, whereby a cotton-like product of apatite having a mean fiber diameter of 3 μm and a mean fiber length of 30 mm was obtained as the pullulan was burned off. It was determined by X-ray diffraction and infrared ray spectrophotometry that the cotton-like end product had been deprived of the hydroxy group.

Example 3

A cotton-like product of hydroxy-apatite having a mean fiber diameter of 15 μm and a mean fiber length of 70 mm after calcining was obtained by the same procedure as the Example 1 except that the amounts of the pullulan particles, hydroxy-apatite particles, and water used were 19%, 31%, and 50% by weight, respectively.

Example 4

A cotton-like product of hydroxy-apatite having a mean fiber diameter of 7 μm and a mean fiber length of 65 mm after calcining was obtained by the same procedure as in Example 1 except that polyvinyl alcohol with a molecular weight of 190,000 was used as a binder.

Example 5

A cotton-like product of hydroxy-apatite having a mean fiber diameter of 3 μm and a mean fiber length of 35 mm was obtained by forming a stream of fibers under the same conditions as in the Example 1 except that a feed solution containing 50 weight% of hydroxy-apatite particles and 15 weight% of pullulan particles was used and that the air was discharged at a velocity of 1200 m/sec.

Example 6

11 weight% of pullulan particles having a mean molecular weight of 200,000, 17 weight% of hydroxy-apatite particles having a particle diameter of 50 to 800Å (5 to 80 nm) and 72 weight% of water were kneaded together to disperse the hydroxy-apatite uniformly in the aqueous pullulan solution. This prepared solution was defoamed and fed into a spinning apparatus as shown in Fig. 2 at room temperature (25°C). The solution was extruded through the nozzles at a pressure of 1.4 kg/cm$^2$ while discharging air through the air gaps at a velocity of 250 m/sec thereby to form a stream of fibers. This fiber stream was heated from opposite sides at a temperature of 400°C by a far-infrared heater (wavelength: 2 to 50 µm) underlying the spinning nozzles to evaporate the water and was blown onto a screen type belt to produce a fibrous product in non-woven fabric form of hydroxy-apatite bonded by pullulan. The resulting non-woven fabric was heated, at a heating rate of 50°C/hr., for calcining at 1100°C for an hour, whereby a non-woven fabric of hydroxy-apatite having a mean fiber diameter of 5 µm and a basis weight of 200 g/m$^2$ was obtained as the pullulan was burned off. It was determined by X-ray diffraction and infrared ray spectrophotometry that the non-woven fabric end product contained substantially the same amount of hydroxy group as the starting material had. Fig. 5 presents micrographs of the fibers of the non-woven fabric before and after the calcining step as observed by electron microscopy.

Example 7

Pullulan particles with a mean molecular weight of 200,000 were dissolved in water to prepare 21 weight% of aqueous solution into which 6 weight% of hydroxy-apatite particles having a particle diameter of 50 to 300Å (5 to 30 nm) was mixed. The solution was then defoamed and fed into the apparatus used in Example 6 at room temperature and extruded at a pressure of 1.4 kg/cm$^2$ while discharging air through the air gaps at a velocity of 500 m/sec, thereby to form a stream of fibers. This fiber stream was heated from opposite sides at a temperature of 400°C by a far-infrared heater (wavelength: 2—50 µm) underlying the spinning nozzles to evaporate the water and was blown onto a screen type belt to produce a fibrous product in non-woven fabric form of hydroxy-apatite bonded by pullulan. The resulting non-woven fabric was heated, at a heating rate of 50°C/hr., for calcining at 1400°C for two hours, whereby a non-woven fabric of apatite having a mean fiber diameter of 3 µm and a basis weight of 200 g/m$^2$ was obtained as the pullulan was burned off. It was determined by X-ray diffraction and infrared ray spectrophotometry that the non-woven fabric product had been deprived of the hydroxy group.

Example 8

A non-woven fabric of hydroxy-apatite having a mean fiber diameter of 15 µm after calcining was obtained by the same procedure as Example 6 except that the amounts of the pullulan particles, hydroxy-apatite particles, and water used were 19%, 31% and 50% by weight, respectively.

Example 9

A non-woven fabric of hydroxy-apatite having a mean diameter of 7 µm after calcining was obtained by the same procedure as Example 6 except that polyvinyl alcohol with a molecular weight of 190,000 was used as a binder.

Example 10

This example was conducted according to Example 6 except that the composition of the viscous dispersion in the feedstock tank 2 comprised 25% of water, 35% of hydroxy-apatite, 30% of calcium phosphate and 10% of pullulan on the basis of weight. The spinning nozzles were 0.3 mm in diameter, the spinning temperature was room temperature (25°C), and the pressure of the air discharged was 300—1000 mm $H_2O$.

Further, the far-infrared heater 9 was 800 to 1000 mm in length, had an output of 4 to 10 kw and provided a heating temperature of 400°C. The non-woven fabric of apatite was heated at a heating rate of 50°C/hr, for calcining at 1100°C for two hours.

The calcined apatite fibers produced in the examples described above had a mean fiber diameter of 3—15 µm and high mechanical strength, that is, high tensile strength and impact resistance.

From the foregoing, it is to be appreciated that since the apatite is solution-spun at a room temperature, rather than being melted at a high temperature, the present invention provides a fibrous product of apatite in cotton-like form and non-woven fabric form which is not deprived of the hydroxy group which would spoil the affinity or compatibility with the living organism, in contrast to the prior art. Consequently, there is no need for after-treatments for imparting "affinity" to the apatite product; the process lends itself to simplification of manufacturing and improved operating efficiency. It is believed that the soluiton spinning at room temperature has been made possible by the discovery that binders such as pullulan have been found which meet the three conditions of non-toxicity to the living organism, water-solubility and decomposability at high temperature without melting. A conventional spinning apparatus used for the so-called melt-blow process or melt spinning process has been so modified that a fibrous product of apatite may be produced by spinning a feed solution through the spinning nozzles to form a stream of fibers, without having to melt the material at a high temperature, heating the stream of fibers to evaporate the water, and blowing the fiber stream onto a collector such as a collector plate or a screen type drum collector conventionally used

for the melt-blow process.

The present invention is the first that has succeeded in obtaining a high strength fibrous apatite by the solution spinning process which has heretofore been considered impossible. A fibrous product, particularly a cotton-like product or non-woven fabric of apatite produced according to this invention is not deprived of the hydroxy group, so the product exhibits much the same affinity with the living organism as the conventional sintered apatite. Furthermore, the hydroxy-apatite content in the fibrous apatite may be adjusted by varying the calcining temperature to control the physical properties such as strength. In addition, owing to its fibrous form, the apatite material according to this invention provides high resistance to impact and superior processing characteristics, so that it may easily be made to conform to the intricate contours of breaks in a bone. Thus, the fibrous apatite of this invention may be used not only for artificial teeth but also for reconstruction of various bones and artificial joints. Further, it may be used for three-dimensional culture media in biotechnology.

**Claims**

1. A fibrous apatite product, such as calcium apatite, characterised in that the product has an intertwined fibrous, cotton-like form and is made of one or more apatites selected from those represented by the general formula:

$$M_{10}(ZO_4)_6X_2$$

wherein M represents Ca, Ba, Mg, Sr, Pb, Cd and Fe, $ZO_4$ represents $PO_4$, $AsO_4$, $VO_4$ and $CO_3$, and X represents F, Cl and OH.

2. The product according to claim 1, wherein M is at least one alkaline earth metal, $ZO_4$ is at least one selected from $PO_4$, $VO_4$ and $CO_3$, and X is OH and/or F.

3. The product according to claim 1, wherein substantially all of the apatite is hydroxy-apatite and wherein M is Ca, $ZO_4$ is $PO_4$ and X is OH.

4. The product according to claim 3, wherein the said hydroxy-apatite contains a calcium phosphate type compound, for example 5 to 95 weight% of hydroxy-apatite and 95 to 5 weight% of the calcium phosphate type compound, and preferably the hydroxy-apatite is 20 weight% or more and the said calcium phosphate type compound is 80 weight% or less.

5. The product according to claim 4, wherein the said hydroxy-apatite is 50 weight% or more and the calcium phosphate type compound is 50 weight% or less.

6. The product according to claim 4 or claim 5, wherein the said calcium phosphate type compound is at least one compound selected from calcium hydrogen phosphate, tri-calcium phosphate and tetra-calcium phosphate.

7. The product according to any of claims 1 to 6, which has a mean fiber diameter of 1 µm to 30 µm and a mean fiber length of 1 mm to 1000 mm.

8. The product according to any of claims 1 to 7, made in the form of a non-woven fabric product, the basis weight of the non-woven fabric preferably being in the range of 5 $g/m^2$ to 200 $g/m^2$.

9. A method of producing the intertwined fibrous, cotton-like apatite product of claim 1, comprising the steps of:

(a) preparing a dispersion of one or more of the selected apatites by dispersing fine apatite particles uniformly in a solution of binder in water, the binder for example being a water soluble high molecular weight compound such as pullulan;

(b) continuously extruding the dispersion through a plurality of spinning orifices while simultaneously stretching the dispersion into a fibrous state with the aid of a high speed air flow to form a stream of fine fibers;

(c) heating the fiber stream to evaporate water in the fibers to dewater the stream to a moisture content of 10% or less;

(d) blowing the so dewatered fiber stream onto a collector means thereby to form the cotton-like product of intertwined fibrous apatite bonded by the binder; and

(e) optionally, if required, calcining the cotton-like product.

10. The method according to claim 9 further including the step of making the cotton-like product of apatite into the form of a non-woven fabric.

11. The method according to claim 9 or claim 10, wherein apatite used is an hydroxy-apatite in which M is Ca, $ZO_4$ is $PO_4$ and X is OH.

12. The method according to claim 9, 10 or 11, wherein said apatite particles are of stick-like form having a mean particle diameter of 50Å (5 nm) to 1 µm.

13. The method according to any of claims 9 to 12, wherein the apatite dispersion further contains fine particles of a calcium phosphate type compound.

14. The method according to any of claims 9 to 13, wherein the fibers are calcined at a temperature in the range of 500°C to 1250°C, for example in the range of 650°C to 1100°C.

**Patentansprüche**

1. Faseriges Apatiterzeugnis, wie Kalziumapatit, dadurch gekennzeichnet, daß das Erzeugnis eine verschlungene faserige, baumwollartige Form besitzt und aus einem oder mehreren Apatiten hergestellt ist, die aus den durch die allgemeine Formel

$$M_{10}(ZO_4)_6X_2$$

dargestellten ausgewählt sind, wobei M für Ca, Ba, Mg, Sr, Pb, Cd und Fe, $ZO_4$ für $PO_4$, $AsO_4$, $VO_4$ und $CO_3$ und X für F, Cl und OH steht.

2. Erzeugnis nach Anspruch 1, bei dem M zumindest ein alkalisches Erdmetall, $ZO_4$ zumindest ein von $PO_4$, $VO_4$ und $CO_3$ ausgewähltes und X OH und/oder F ist.

3. Erzeugnis nach Anspruch 1, bei dem im

wesentlichen der gesamte Apatit Hydroxylapatit ist und wobei M=Ca, $ZO_4$=$PO_4$ und X=OH.

4. Erzeugnis nach Anspruch 3, bei dem der Hydroxylapatit eine Kalziumphosphatverbindung, zum Beispiel 5 bis 95 Gew.% Hydroxylapatit und 95 bis 5 Gew.% der Kalziumphosphatverbindung, enthält und vorzugsweise der Hydroxylapatit 20 Gew.% oder mehr und die Kalziumphosphatverbindung 80 Gew.% oder weniger beträgt.

5. Erzeugnis nach Anspruch 4, bei dem der Hydroxylapatit 50 Gew.% oder mehr und die Kalziumphosphatverbindung 50% oder weniger beträgt.

6. Erzeugnis nach Anspruch 4 oder Anspruch 5, bei dem die Kalziumphosphatverbindung zumindest eine aus Kalziumhydrogenphosphat, Trikalziumphosphat und Tetrakalziumphosphat ausgewählte Verbindung ist.

7. Erzeugnis nach einem der Ansprüche 1 bis 6, das einen mittleren Faserdurchmesser von 1 µm bis 30 µm und eine mittlere Faserlänge von 1 mm bis 1000 mm besitzt.

8. Erzeugnis nach einem der Ansprüche 1 bis 7, hergestellt in Form eines Non-Woven-Erzeugnisses, wobei das Flächengewicht des Non-Wovens vorzugsweise im Bereich von 5 $g/m^2$ bis 200 $g/m^2$ liegt.

9. Verfahren zur Herstellung des verschlungenen faserigen, baumwollartigen Apatiterzeugnisses nach Anspruch 1, mit den Schritten, daß

a) eine Dispersion eines oder mehrerer der ausgewählten Apatiten hergestellt wird, indem feine Apatitpartikel gleichmäßig in einer Lösung eines Bindemittels in Wasser dispergiert werden, wobei das Bindemittel zum Beispiel eine wasserlösliche Verbindung hohen Molekulargewichts wie Pullulan ist,

b) die Dispersion kontinuierlich durch eine Mehrzahl von Spinnöffnungen extrudiert wird, während gleichzeitig die Dispersion in einen faserigen Zustand mit Hilfe eines Hochgeschwindigkeitsluftstroms zur Bildung eines Stroms feiner Fasern gestreckt wird,

c) der Faserstrom zum Verdampfen von Wasser in den Fasern zur Entwässerung des Stroms auf einen Feuchtigkeitsgehalt von 10% oder weniger erwärmt wird,

d) der so entwässerte Faserstrom auf eine Sammeleinrichtung geblasen wird, um dadurch das baumwollartige Erzeugnis aus durch das Bindemittel gebundenem verschlungenem faserigem Apatit zu bilden, und

e) wahlweise, falls erforderlich, das baumwollartige Erzeugnis gebrannt wird.

10. Verfahren nach Anspruch 9, mit dem weiteren Schritt, daß das baumwollartige Erzeugnis aus Apatit in der Form eines Non-Wovens hergestellt wird.

11. Verfahren nach Anspruch 9 oder Anspruch 10, bei dem der verwendete Apatit ein Hydroxylapatit ist, in dem M=Ca, $ZO_4$=$PO_4$ und X=OH.

12. Verfahren nach Anspruch 9, 10 oder 11, bei dem die Apatitpartikel eine stabähnliche Form mit einem mittleren Partikeldurchmesser von 50Å (5 nm) bis 1 µm besitzen.

13. Verfahren nach einem der Ansprüche 9 bis 12, bei dem die Apatitdispersion ferner feine Partikel einer Kalziumphosphatverbindung enthält.

14. Verfahren nach einem der Ansprüche 9 bis 13, bei dem die Fasern mit einer Temperatur im Bereich von 500°C bis 1250°C, beispielsweise im Bereich von 650°C bis 1100°C, gebrannt werden.

**Revendications**

1. Produit fibreux à base d'apatite, notamment d'apatite calcique, caractérisé en ce qu'il à une forme cotonneuse de fibres entre lacées et en ce qu'il est fait d'une ou de plusieurs apatites choisies parmi celles qui sont représentées par la formule générale

$$M_{10}(ZO_4)_6X_2$$

dans laquelle M représente Ca, Ba, Mg, Sr, Pb, Cd et Fe, $ZO_4$ représente $PO_4$, $AsO_4$, $VO_4$ et $CO_3$, et X représente F, Cl et OH.

2. Produit selon la revendication 1, dans lequel M est au moins un métal alcalino-terreux, $ZO_4$ est choisi au moins parmi $PO_4$, $VO_4$ et $CO_3$, et X est mis pour OH et/ou F.

3. Produit selon la revendication 1, dans lequel la totalité pratiquement de l'apatite est de l'hydroxyapatite et dans lequel M est mis pour Ca, $ZO_4$ est mis pour $PO_4$ et X est mis pour OH.

4. Produit selon la revendication 3, dans lequel ladite hydroxy-apatite contient un composé du type phosphate de calcium, par exemple de 5 à 95% en poids d'hydroxy-apatite et de 95 à 5% en poids du composé du type phosphate de calcium, l'hydroxy-apatite étant de préférence présente à raison de 20% en poids ou plus et ledit composé du type phosphate de calcium à raison de 80% en poids ou moins.

5. Produit selon la revendication 4, dans lequel l'hydroxy-apatite est présente à raison de 50% en poids ou plus et le composé du type phosphate de calcium à raison de 50% en poids ou moins.

6. Produit selon la revendication 4 ou 5, dans lequel ledit composé du type phosphate de calcium est au moins un composé choisi parmi l'hydrogénophosphate de calcium, le phosphate tricalcique et le phosphate tétracalcique.

7. Produit selon l'une quelconque des revendications 1 à 6 qui a un diamètre moyen des fibres de 1 µm à 30 µm et une longueur moyenne des fibres de 1 mm à 1000 mm.

8. Produit selon l'une quelconque des revendications 1 à 7, fabriqué sous la forme d'un produit textile non tissé, le poids de base du non-tissé se situant de préférence dans la gamme de 5 $g/m^2$ à 200 $g/m^2$.

9. Procédé de fabrication du produit d'apatite cotonneux à fibres entrelacées selon la revendication 1, comprenant les étapes consistant à

(a) préparer une dispersion d'une ou de plusieurs des apatites choisies en dispersant uniformément de fines particules d'apatite dans une solution de liant dans l'eau, le liant étant par

exemple un composé hydrosoluble de poids moléculaire élevé tel que le pullulane;

(b) extruder de façon continue la dispersion à travers une multiplicité d'orifices de filage, tout en étirant simultanément la dispersion à l'état de fibres à l'aide d'un courant d'air à haute vitesse pour former un courant de fibres fines;

(c) chauffer le courant de fibres pour évaporer l'eau contenue dans les fibres et déshydrater le courant jusqu'à une teneur en humidité de 10% ou moins;

(d) souffler le courant de fibres ainsi déshydraté sur des moyens collecteurs de manière à former le produit cotonneux d'apatite en fibres entrelacées unies par le liant; et

(e) facultativement, calciner au besoin le produit cotonneux.

10. Procédé selon la revendication 9, comprenant en outre l'étape consistant à mettre le produit cotonneaux d'apatite sous la forme d'un non-tissé.

11. Procédé selon la revendication 9 ou 10, dans lequel l'apatite utilisée est une hydroxyapatite dans laquelle M est mis pour Ca, $ZO_4$ pour $PO_4$ et X pour OH.

12. Procédé selon l'une quelconque des revendications 9 à 11, dans lequel lesdites particules d'apatite ont la forme de bâtonnets ayant un diamètre moyen de particules de 50Å (5 nm) à 1 µm.

13. Procédé selon l'une quelconque des revendications 9 à 12, dans lequel la dispersion d'apatite contient en outre de fines particules d'un composé du type phosphate de calcium.

14. Procédé selon l'une quelconque des revendications 9 à 13, dans lequel les fibres sont calcinées à une température dans la gamme de 500°C à 1250°C, par exemple dans la gamme de 650°C à 1100°C.

Fig. 1

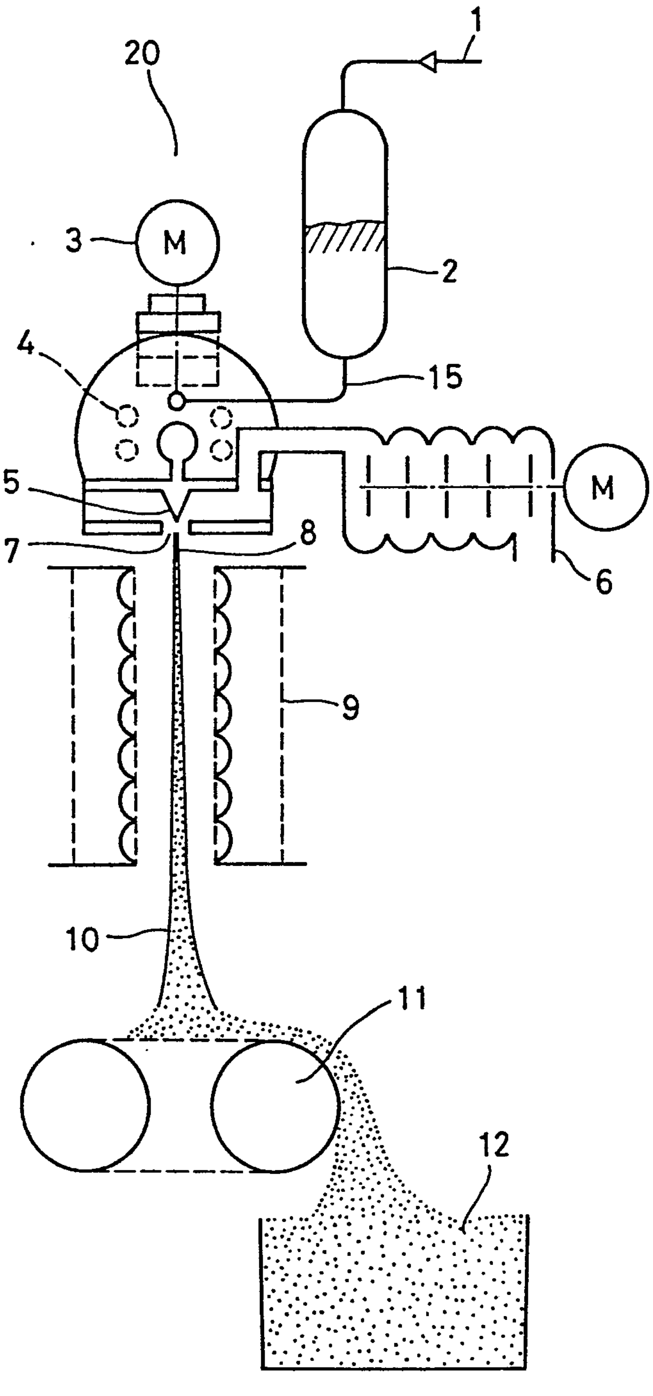

Fig. 2

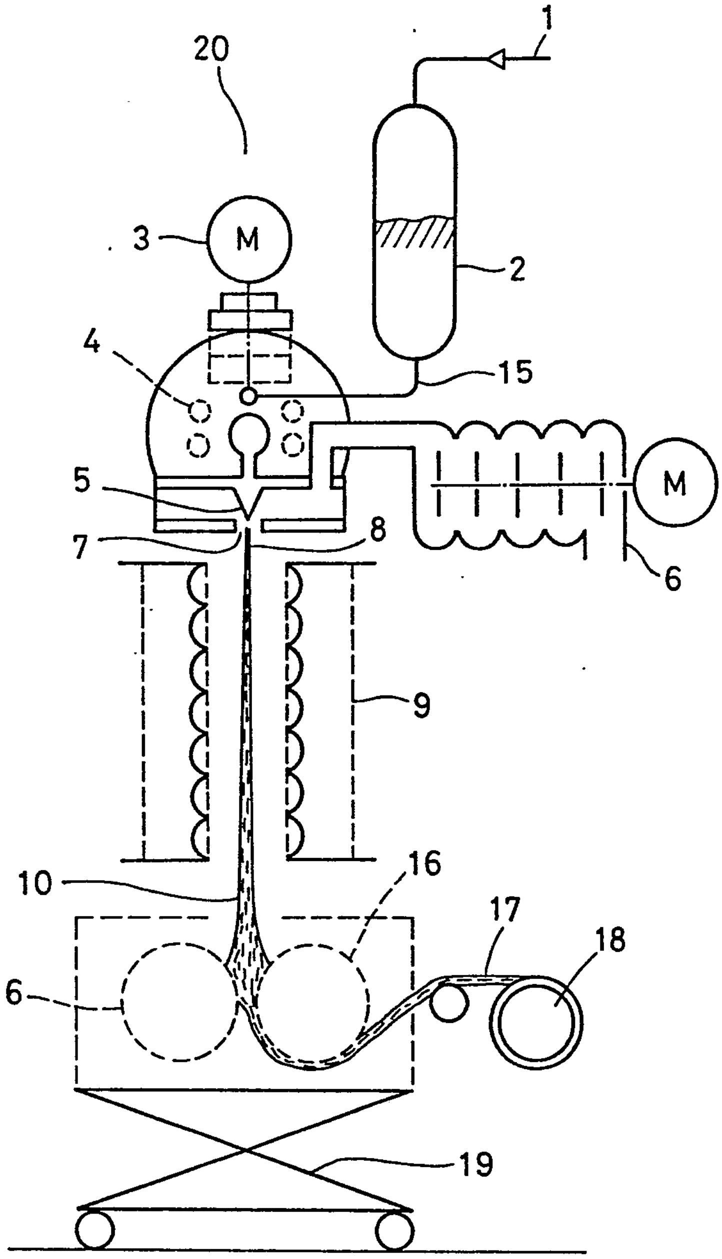

Fig. 3

o : $Ca_5(PO_4)_3(OH)$

Δ : $\beta-Ca_3(PO_4)_2$

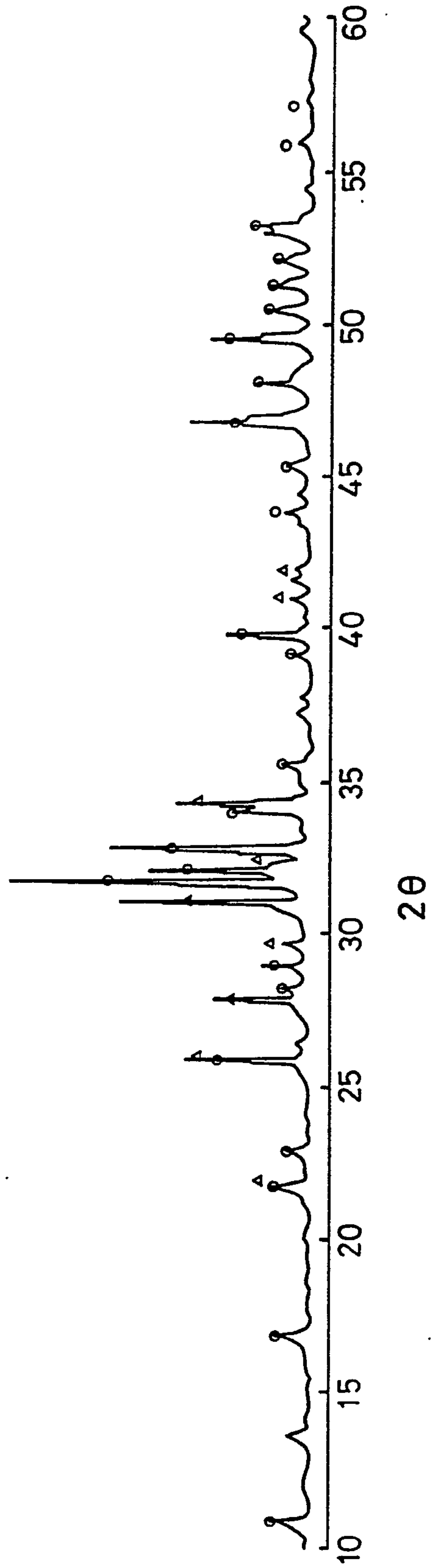

Fig. 4

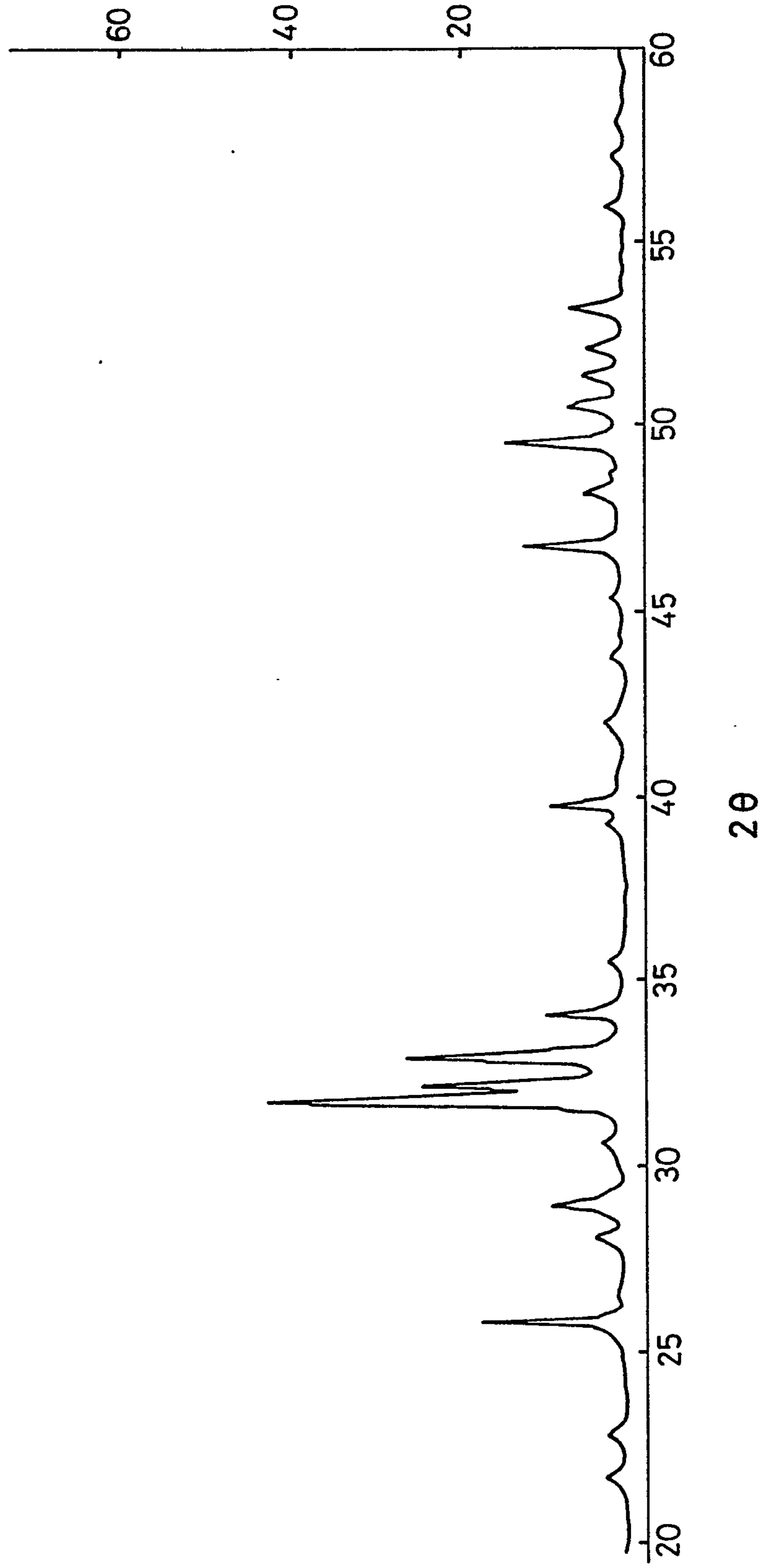

*FIG. 5.*

ELECTRON MICROGRAPHS OF APATITE FIBERS

PRIOR TO CALCINING

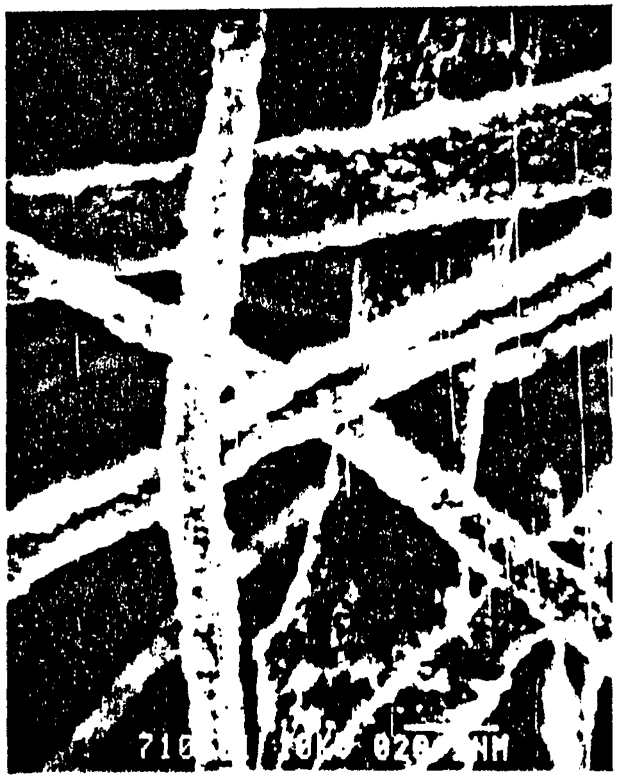

MAGNIFICATION x 4000

AFTER TO CALCINING

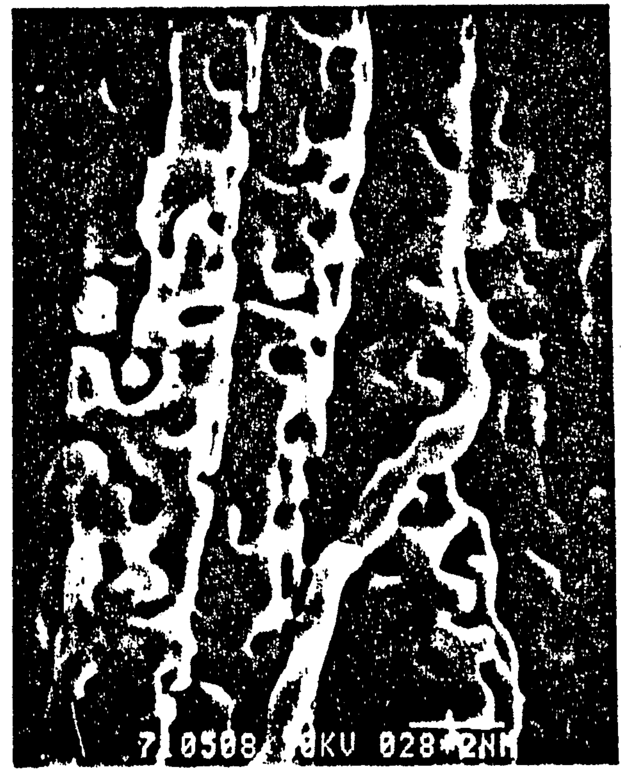

MAGNIFICATION x 4000